(19) Europäisches Patentamt
European Patent Office
Office européen des brevets

(11) Veröffentlichungsnummer: **0 089 011**
**B1**

(12) # EUROPÄISCHE PATENTSCHRIFT

(45) Veröffentlichungstag der Patentschrift: **10.07.85**

(51) Int. Cl.⁴: **C 07 C 120/00**, C 07 C 121/34, C 07 C 121/76, C 07 D 333/24

(21) Anmeldenummer: **83102344.5**

(22) Anmeldetag: **10.03.83**

(54) **Verfahren zur Herstellung von 3-Oxonitrilen.**

(30) Priorität: **16.03.82 DE 3209472**

(43) Veröffentlichungstag der Anmeldung: **21.09.83 Patentblatt 83/38**

(45) Bekanntmachung des Hinweises auf die Patenterteilung: **10.07.85 Patentblatt 85/28**

(84) Benannte Vertragsstaaten: **AT BE CH DE FR GB IT LI LU NL**

(56) Entgegenhaltungen: **GB - A - 787 858**

(73) Patentinhaber: **Degussa Aktiengesellschaft, Weissfrauenstrasse 9, D-6000 Frankfurt am Main 1 (DE)**

(72) Erfinder: **Drauz, Karlheinz, Dr., Dipl.-Chem., Flurstrasse 5, D-6463 Freigericht 1 (DE)**
Erfinder: **Kleemann, Axel, Dr., Dipl.-Chem., Greifenhagenstrasse 9, D-6450 Hanau am Main 9 (DE)**
Erfinder: **Wolf-Heuss, Elisabeth, Dr., Dipl.-Chem., Bismarckweg 35, D-6950 Mosbach (DE)**

EP 0 089 011 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein neues Verfahren zur Herstellung von 3-Oxonitrilen durch Kondensation von Carbonsäureestern mit Carbonsäurenitrilen sowie neue 3-Oxonitrile. Es ist bekannt, 3-Oxonitrile durch Dimerisierung von Carbonsäurenitrilen in Gegenwart starker Basen nach Verseifung der intermediär gebildeten Iminonitrile in Ausbeuten von maximal 80% herzustellen (Houben-Weyl, Band VII/2a, S. 515). Dieses Verfahren läßt sich jedoch nur zur Herstellung solcher 3-Oxonitrile der allgemeinen Formel I anwenden, bei denen die mit der C=O-Gruppe und mit der —CH-Gruppe verbundenen Reste $R_1$ und $R_2$ gleich sind.

Geht man von verschiedenartigen Nitrilen aus, erhält man Produktgemische.

Es ist weiter bekannt, daß man durch Kondensation von Carbonsäureestern mit Carbonsäurenitrilen in Gegenwart starker Basen direkt die 3-Oxonitrile erhalten kann. Stark CH-acide Carbonsäurenitrile, wie Benzylcyanid, lassen sich mit Alkoholat kondensieren. Die Ausbeuten liegen zwischen 65 und 70%, bezogen auf den eingesetzten Carbonsäureester.

Die Acylierung der weniger aciden aliphatischen Nitrile gelingt erst bei erhöhten Temperaturen. So verschlechtert sich die Ausbeute bei der Herstellung von 2-Benzoylpropionitril auf Grund von unerwünschten Nebenreaktionen auf 53% (Houben-Weyl VIII, S. 573).

Ferner ist noch bekannt, daß die Kondensation aliphatischer Nitrile mit Carbonsäureestern nur mit feinverteiltem Natriumamid in flüssigem Ammoniak in präparativ befriedigenden Ausbeuten durchführbar ist (Houben-Weyl VIII, S. 574, J. Am. Chem. Soc. 68, 760 (1946)).

In Übereinstimmung mit diesen Angaben ergibt die Kondensation von 2-Methoxybenzoesäuremethylester mit Acetnitril unter Verwendung von Natriumamid/flüssigem Ammoniak in 84%iger Ausbeute 2-Methoxybenzoylacetonitril; dagegen führt dieselbe Umsetzung bei Verwendung von Natriumhydrid in Benzol nur zu einer 27,4%igen Reaktionsausbeute (Bull. Soc. Japan 35, 1869 (1962)).

Ferner ist die Umsetzung von Propionsäureethylester mit Acetonitril unter Verwendung von nur 50gew.-%igem Natriumhydrid in Öl bekannt. Dabei wird das Natriumhydrid in Benzol bei Siedetemperatur zuerst mit dem Acetonitril versetzt, und dann der Carbonsäureester zugetropft. Bei dieser Verfahrensweise besteht die Gefahr der Selbstkondensation des Acetonitril. Deshalb wird das 3-Oxonitril in nur 52%iger Ausbeute erhalten (Bull. Soc. Chim. France 1971, 2195)).

Diese Ausbeuten sind völlig unzureichend und gestatten keine großtechnische Synthese der 3-Oxonitrile.

Weitere Verfahren zur Darstellung von 3-Oxonitrilen sind die Umsetzung von Chlorsulfonylisocyanat mit Ketonen und nachfolgender Behandlung der N-Chlorsulfonyl-3-oxoamide mit Dimethylformamid unter Freisetzung der 3-Oxonitrile (Synthesis 1973, 682), ebenso wie die Umsetzung von Enaminen mit Chlorcyan (J. Am. Chem. Soc. 81, 5400 (1959)).

Beide Methoden sind präparativ sehr aufwendig und erfordern erhebliche Sicherheitsmaßnahmen infolge der Gefährlichkeit der Einsatzstoffe. Zudem liegen die Reaktionsausbeuten bei maximal 50%, so daß eine wirtschaftliche Synthese nicht durchführbar ist.

Für einzelne 3-Oxonitrile sind auch spezielle Synthesen bekannt geworden. So wird z. B. das Pivaloylacetonitril aus Pinakolon durch Chlorierung und Umsetzung des Monochlorpinakolons mit einem Alkalimetallcyanid erhalten (DE-OS 2 819 264).

Dieses Verfahren ist mehrstufig und erfordert den Umgang mit extrem giftigen Cyaniden. Außerdem ist bekannt, daß α-Chlorketone, wie es das α-Chlorpinakolon darstellt, gefährliche Reizstoffe sind.

Es wurde nun gefunden, daß man 3-Oxonitrile der allgemeinen Formel I,

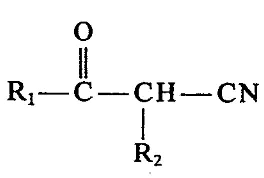

(I)

in der $R_1$ einen jeweils gegebenenfalls substituierten, tertiären Alkyl-, Cycloalkyl-, aromatischen oder heteroaromatischen Rest bedeutet, und $R_2$ für einen geraden oder verzweigten, gegebenenfalls substituierten Alkylrest, mit 1 bis 4 C-Atomen, einen gegebenenfalls substituierten Aryl- oder Heteroarylrest, oder für ein Wasserstoffatom steht, durch Umsetzung eines Carbonsäureesters der allgemeinen Formel II,

$$R_1{-}COOR_3 \qquad (II)$$

in der $R_3$ für den Methyl- oder Äthylrest steht, mit einem Carbonsäurenitril der allgemeinen Formel III,

$$R_2CH_2C{\equiv}N \qquad (III)$$

wobei $R_1$ und $R_2$ die oben angegebenen Bedeutungen haben, in einem inerten Lösungsmittel in

Gegenwart von Natriumhydrid und in Gegenwart eines Öles herstellen kann, wenn man das Natriumhydrid in Form einer 70—80%igen Suspension in Weißöl einsetzt, und dieses zusammen mit dem Ester gemäß der allgemeinen Formel II vorlegt.

Das erfindungsgemäße Verfahren ist ohne die oben aufgeführten Nachteile durchführbar und eröffnet erstmalig eine allgemein anwendbare Herstellungsmethode für 3-Oxonitrile. Es ist einstufig, ergibt hohe Ausbeuten und liefert Produkte in hoher Reinheit.

Die Verbindungen der allgemeinen Formel I, von denen einige neu sind, sind wertvolle Zwischenprodukte für die Herstellung von 3-Ketocarbonsäureestern und -amiden, Heterocyclen und Pestiziden. Beispiel ist das Pivaloylacetonitril, ein wichtiges Zwischenprodukt für ein Isoxazolherbizid. (DE-OS 2 436 179 und 2 819 264).

Unter den Carbonsäureestern der allgemeinen Formel II sind jene bevorzugt, die nicht enolisierbar sind. Unter den angeführten Bedeutungen für das Symbol $R_1$ steht der Begriff tert.-Alkylgruppe für solche Reste, deren in $\alpha$-Stellung befindliches C-Atom kein Wasserstoffatom trägt. Sie können vorzugsweise 4—12 C-Atome aufweisen und ebenso wie die aromatischen Reste zum Beispiel durch Gruppen substituiert sein, die gegenüber Natriumhydrid inert sind. Hierzu gehören insbesondere Halogenatome, Alkoxy- und Alkylmercaptogruppen.

Die Cycloalkylreste können in gleicher Weise substituiert sein und umfassen vorzugsweise 3 bis 6 Glieder.

Die heteroaromatischen Reste, die für $R_1$ und $R_2$ stehen können und insbesondere 5 oder 6 Glieder aufweisen, können vorzugsweise O-, S- oder N-Atome besitzen. Sie können anneliert oder kondensiert sein.

Geeignete Carbonsäureester sind beispielsweise die

Methyl- bzw. Ethylester der Pivalinsäure, 2,2-Dimethylbuttersäure, 2,2-Dimethylvaleriansäure, 2,2-Dimethylhexansäure, 1-Methylcyclopropancarbonsäure, 1-Methylcyclobutancarbonsäure, 1-Methylcyclopentancarbonsäure, 1-Methylcyclohexancarbonsäure, 2,2-Dichlor-1-methylcyclopropancarbonsäure, Benzoesäure, 2-Methyl-, 3-Methyl-, 4-Methyl-, 2,4-Dimethyl-, 2-Fluor-, 3-Fluor-, 4-Fluor-, 2-Chlor-, 3-Chlor-, 4-Chlor-, 2,4-Dichlor-, 3,4-Dichlor-, 3,5-Dichlor-, 2-Methoxy-, 3-Methoxy-, 4-Methoxy-, 3,4-Dimethoxy-, 3,4,5-Trimethoxy-, 2-Trifluormethylbenzoesäure, Furan-2-carbonsäure, Furan-3-carbonsäure, 5-Brom-2-methyl-furan-3-carbonsäure, Thiophen-2-carbonsäure, Thiophen-3-carbonsäure, 5-Methyl-thiophen-2-carbonsäure.

Pro Mol des eingesetzten Carbonsäureesters werden vorteilhaft 1,5 bis 2,1 Mol, vorzugsweise 2 Mol, des Natriumhydrids eingesetzt.

Als inerte Lösungsmittel verwendet man zweckmäßigerweise Ether bzw. aliphatische bzw. aromatische Kohlenwasserstoffe, wie z. B. Cyclohexan, Benzol oder Toluol.

Das Natriumhydrid und der Carbonsäureester werden im Lösungsmittel gelöst bzw. suspendiert und erwärmt. Es kann dabei zweckmäßig sein, in einer Inertgasatmosphäre z. B. unter Stickstoff oder Argon zu arbeiten. In diese erwärmte, stark gerührte Suspension wird dann das Carbonsäurenitril der allgemeinen Formel II zugetropft.

In der allgemeinen Formel III kann $R_2$ die oben angegebenen Bedeutungen haben, wobei im Falle der aromatischen Reste und der Alkylreste die gleichen Substitutionen, wie unter $R_1$ angegeben, möglich sind.

Als Carbonsäurenitrile finden beispielsweise Verwendung:

Acetonitril, Propionitril, Butyronitril, Valeronitril, Hexansäurenitril, Benzylcyanid, 2-Chlorbenzylcyanid, 4-Chlorbenzylcyanid, 4-Fluorbenzylcyanid, 2-Methylbenzylcyanid, 4-Methylbenzylcyanid, Thiophen-2-acetonitril, Thiophen-3-acetonitril, Furan-3-acetonitril, 5-Chlorthiophen-2-acetonitril.

Die Menge des bei der Reaktion eingesetzten Carbonsäurenitrils der allgemeinen Formel III liegt vorteilhaft zwischen 1,0 und 2,1 Mol pro Mol eingesetzten Carbonsäureesters. Bevorzugt wird eine Menge zwischen 1,5 und 2,0 Mol Nitril pro Mol Ester.

Die Reaktion kann in einem Temperaturbereich zwischen 50° und 110°C durchgeführt werden. Vorteilhaft wird ein Bereich zwischen 60° und 100°C, vorzugsweise zwischen 80° und 95°C eingehalten. Der Reaktionsbeginn kann durch Zugabe einer katalytischen Menge Alkohol (Methanol, Ethanol etc.) erleichtert werden. Er wird durch das Entweichen von Wasserstoff angezeigt. Im Verlauf der Reaktion kann die Reakionsgeschwindigkeit (angezeigt durch die Menge pro Zeiteinheit entweichenden $H_2$) durch die Zugabe des Nitrils gut gesteuert werden.

Das Reaktionsende wird durch die Beendigung der Wasserstoffentwicklung und durch den vollständigen Umsatz des NaH in der Reaktionslösung angezeigt.

Die Aufarbeitung erfolgt in an sich bekannter Weise beispielsweise durch Ausrühren der Reaktionsmischung (Suspension) mit einer genügend großen Menge Wasser, so daß sich der in der Reaktionsmischung befindliche Feststoff unter Bildung zweier klarer Phasen auflöst. Die wäßrige Phase wird

abgetrennt, die organische Phase gegebenenfalls mit weiterem Wasser ausgerührt, die Wasserphasen vereinigt, abgekühlt und unter Kühlung mit wäßriger Mineralsäure auf einen pH-Wert zwischen 1 und 5 eingestellt. Dabei scheidet sich das 3-Oxonitril in fester Form oder als Öl ab.

Die Isolierung des Produktes erfolgt durch Absaugen des Feststoffes, beziehungsweise Abtrennung des Öls.

Die Produkte werden, gegebenenfalls nach einem Nachwaschen mit Wasser, getrocknet und — sofern nötig — durch Umkristallisieren oder Fraktionieren weiter gereinigt.

Löst sich der in der Reaktionslösung befindliche Niederschlag bei Zugabe von Wasser nur schwer, kann es zweckmäßig sein, diesen Niederschlag abzusaugen, mit dem verwendeten Lösungsmittel nachzuwaschen, und das Produkt zur Neutralisation unter sehr starkem Rühren in eine wäßrige Mineralsäure einzutragen. Die Säuremenge wird zweckmäßigerweise so gewählt, daß nach vollständiger Reaktion ein pH-Wert zwischen 1 und 5 in der erhaltenen wäßrigen Lösung vorliegt. Die bevorzugte Mineralsäure ist Salzsäure.

Gegebenenfalls kann es günstig sein, bei dieser Verfahrensweise auch Essigsäure oder ein Gemisch Essigsäure/Mineralsäure einzusetzen. Man erhält dann die 3-Oxonitrile in fester bzw. flüssiger Form, die, wie vorstehend beschrieben, gereinigt werden können. Zweckmäßigerweise sollte bei der Aufarbeitung die Behandlung mit der Säure bei einer Temperatur durchgeführt werden, die 10° C, vorzugsweise 0° C, nicht übersteigt.

Bei dem als Suspensionsmittel für das Natriumhydrid verwendeten Weißöl handelt es sich um das Handelsprodukt Shell Oudina 15® der Firma Shell. Es besteht zu 65% aus Paraffinen und zu 35% aus Naphthenen in einer DAB 8-Qualität. Es hat einen Siedepunkt von 337 bis 370° C/760 Torr.

Die Erfindung wird durch nachfolgende Beispiele erläutert:

## Beispiel 1

### Pivaloylacetonitril

55 g Natriumhydrid (als 80gew.-%ige Suspension in Weißöl) werden in 500 ml trockenem Toluol aufgeschlämmt, 106 g (0,914 Mol) Pivalinsäuremethylester zugegeben und auf 85° C erhitzt. Dann werden unter starkem Rühren 77 g (1,87 Mol) Acetonitril innerhalb von 4 Stunden zugetropft. Bei 85° C wird anschließend bis zum Ende der Wasserstoffentwicklung weitergerührt. Die dickflüssige Reaktionsmasse wird auf Raumtemperatur abgekühlt, mit 700 ml Wasser versetzt, 30 Minuten kräftig gerührt und die beiden Phasen im Scheidetrichter getrennt. Die wäßrige Phase wird bei 0° C mit einer 31gew.-%igen wäßrigen Salzsäure auf pH 1—2 angesäuert. Das ausgefallene Pivaloylacetonitril wird abgesaugt, mit Eiswasser neutral gewaschen und bei 25 Torr und 40° C bis zur Gewichtskonstanz getrocknet. Man erhält 106 g (93% d. Th.) analysenreines Pivaloylacetonitril mit einem Schmelzpunkt von 65—68° C.

Analyse: $C_7H_{11}NO$ (125,0)

| | | | |
|---|---|---|---|
| Ber.: | C 67,29 | H 8,93 | N 11,0 |
| Gef.: | C 67,17 | H 8,86 | N 11,19 |

## Beispiel 2

### Pivaloylacetonitril

In 480 l durch Azeotropdestillation entwässertes Toluol werden bei 50° C unter einer Stickstoffatmosphäre nacheinander 67,76 kg (584 Mol) Pivalinsäuremethylester und 35 kg einer 80gew.-%igen Suspension von Natriumhydrid in Weißöl gegeben. Die Aufschlämmung wird auf 85° C erwärmt und innerhalb von 6 Stunden mit 48,88 kg (1192 Mol) Acetonitril versetzt. Die Reaktionsmischung wird bis zur Beendigung der Wasserstoffentwicklung noch 1,5 Stunden bei 85° C gerührt, auf 25° C abgekühlt und mit 500 l Wasser ausgerührt. Die wäßrige Phase wird abgetrennt, auf 0° C abgekühlt und mit 130 l konzentrierter Salzsäure unter Kühlung und Rühren auf pH 2 angesäuert. Das ausgefallene Produkt wird abgetrennt, mit Wasser neutral gewaschen und im Vakuum bei 50 Torr und 40° C getrocknet.

Man erhält 68,6 kg (94% d. Th.) Pivaloylacetonitril mit einem Schmelzpunkt von 65—68° C.

Beispiel 3

1'-Methylcyclopropanoylacetonitril

128,2 g (1 Mol) 1-Methylcyclopropancarbonsäuremethylester und 60 g (1 Mol) Natriumhydrid 80gew.-%ig werden in 750 ml trockenem Toluol auf 80°C erwärmt und unter starkem Rühren 82,1 g (2 Mol) Acetonitril innerhalb 1 Stunde zugetropft. Bei dieser Temperatur wird bis zur Beendigung der Wasserstoffentwicklung weitergeführt. Nach Abkühlen auf Raumtemperatur wird die Reaktionsmasse mit insgesamt 1 l Wasser versetzt, ausgerührt und nach Phasentrennung die wäßrige Phase mit Salzsäure auf pH 1,5 eingestellt. Die Temperatur wird dabei in einem Bereich zwischen 0° und +5°C gehalten.

Das sich abscheidende Öl wird abgetrennt, die Wasserphase mit 500 ml Methylenchlorid extrahiert, die organischen Phasen vereinigt, über Natriumsulfat getrocknet und das Lösungsmittel abdestilliert.

Der ölige Rückstand wird im Vakuum-fraktioniert. Bei 130—132°C/20 mm gehen 104,9 g (85% d. Th.) 1-Methylcyclopropanoylacetonitril über.

Analyse: $C_7H_9NO$ (123,16)

| | |
|---|---|
| Ber.:C 68,30H 7,40 | N 11,40 |
| Gef.:C 68,31H 7,44 | N 11,52 |

$^1$H-NMR ($CDCl_3$) =
$\delta$=3,70 (S, 2H) $CH_2$—CN; 1,45 (S, 3H) $CH_3$; 1,4—0,65 ppm (m, 5H) Cyclopropyl.

Beispiel 4

(2',2'-Dichlor-1'-methyl)cyclopropanoylacetonitril

183 g (1 Mol) 2,2-Dichlor-1-methylcyclopropancarbonsäuremethylester und 60 g (2 Mol) 80gew.-%iges Nariumhydrid werden in 750 ml trockenem Toluol mit 82,1 g (2 Mol) Acetonitril, wie im Beispiel 3 beschrieben, umgesetzt.

Nach der Destillation erhält man 117,6 g (61,2% d. Th.) (2,2-Dichlor-1-methyl)cyclopropanoylacetonitril mit einem Siedepunkt von 102—103°C bei 0,5 Torr.

Analyse: $C_7H_7Cl_2NO$ (192,05)

| | |
|---|---|
| Ber.:C 43,77H 3,67 | N 7,29Cl 36,92 |
| Gef.:C 44,18H 4,21 | N 7,63Cl 36,91 |

$^1$H—NMR ($CDCl_3$):
$\delta$=4,95, 4,90, (S, 2H) $CH_2$—CN; 1,95 (AB, 2H) $C(Cl_2)—CH_2$; 1,70 ppm (S, 3H) C—$CH_3$.

Beispiel 5

2-Thiophenoylacetonitril

78,1 g, (0,5 Mol) Thiopen-2-carbonsäureethylester und 30 g (1 Mol) Natriumhydrid (80gew.-%ige Suspension in Weißöl) werden in 500 ml trockenem Toluol mit 41,5 g (1 Mol) Acetonitril, wie in Beispiel 1 beschrieben, umgesetzt. Man erhält 70,0 g (92,6% d. Th.) 2-Thiophenoylacetonitril mit einem Schmelzpunkt von 110°C.

Analyse: $C_7H_5NOS$ (151,18)

| | |
|---|---|
| Ber.:C 55,61H 3,33 | N 9,27S 21,21 |
| Gef.:C 55,49H 3,46 | N 9,11S 21,05 |

$^1$H—NMR (DMSO/$d_6$):
$\delta$=8,1—7,0 (m, 3H) H Thiophen, 4,33 ppm (S, 2H) $CH_2$ CN.

Beispiel 6

2-Furanoylacetonitril

63,05 g (0,5 Mol) Furan-2-carbonsäureethylester und 30,0 g (1 Mol) Natriumhydrid (80gew.-%ige Suspension in Weißöl) werden in 500 ml trockenem Toluol mit 41 g (1 Mol) Acetonitril unter Zugabe von 1 ml Methanol bei 90° C umgesetzt. Nach 3stündiger Reaktionszeit wird das Toluol abdestilliert, der Rückstand mit 500 ml Wasser ausgerührt, mit Salzsäure auf pH 1,5 angesäuert und das ausgefallene Produkt abgesaugt und aus Methanol umkristallisiert. Man erhält 51,3 g (76% d. Th.) 2-Furanoylacetonitril vom Schmelzpunkt 74—75° C.

Analyse: $C_7H_5NO_2$ (135,12)

| | | | |
|---|---|---|---|
| Ber.: | C 62,33 | H 3,73 | N 10,36 |
| Gef.: | C 61,86 | H 3,48 | N 10,11 |

$^1$H—NMR ($CDCl_3$):
$\delta$=7,69 (S, 1H), 7,38 (d, 1H); 6,63 (m, 1H) $H_{Furan}$; 4,0 ppm (S, 2H) $CH_2$—CN.

Beispiel 7

Benzoylacetonitril

70 g (0,5 Mol) Benzoesäureethylester werden mit 30 g (1 Mol) Natriumhydrid (80gew.-%ige Suspension in Weißöl) in 500 ml trockenem Toluol auf 75—80° C erhitzt. Innerhalb von 2 Stunden werden 41 g (1 Mol) Acetonitril zugetropft und bis zum Ende der Wasserstoffentwicklung bei 85° C gerührt. Die auf Raumtemperatur abgekühlte Reaktionsmischung wird abgesaugt und der Feststoff in eine Mischung aus 9 Teilen Eisessig und 1 Teil 31gew.-%ige Salzsäure bei 0° C eingerührt, und diese Reaktionsmischung anschließend auf 700 ml Eis gegossen.

Der ausgefallene Feststoff wird abgesaugt, mit Wasser neutral gewaschen und bei 50 Torr/50° C bis zur Gewichtskonstanz getrocknet.

Man erhält 64,5 g (89% d. Th.) Benzoylacetonitril vom Schmelzpunkt 80—82° C.

Beispiel 8

4-Methoxybenzoylacetonitril

166,2 g (1 Mol) 4-Methoxybenzoesäuremethylester und 60 g (2 Mol) Natriumhydrid (80gew.-%ige Suspension in Weißöl) werden in 750 ml trockenem Toluol auf 65° C erhitzt. Innerhalb von 2 Stunden werden 82,1 g (2 Mol) Acetonitril bei 85° C zugetropft und danach weitere 20 Stunden bei 90° C gerührt. Der Niederschlag wird abgesaugt, unter Kühlung langsam in 200 ml Eisessig eingerührt und anschließend auf 1 l Eiswasser gegeben. Der ausgefallene Kristallbrei wird abgesaugt, mit Wasser nachgewaschen und aus Aceton umkristallisiert. Man erhält so 149 g (85% d. Th.) 2-Methoxybenzoylacetonitril mit einem Schmelzpunkt von 127—129° C.

Beispiel 9

2,4,4-Trimethyl-3-oxopentannitril

234,6 g (2 Mol) Pivalinsäuremethylester und 120 g (2 Mol) Natriumhydrid (80gew.-%ige Suspension in Weißöl) werden in 1500 ml trockenem Toluol auf 90°C erwärmt. Bei dieser Temperatur werden 233,3 g (2 Mol) Propionitril nach Zusatz von 1 ml Methanol innerhalb von 2,5 Stunden zugetropft. Nach Beendigung der Wasserstoffentwicklung wird die Suspension mit insgesamt 1200 ml Wasser extrahiert und nach Phasentrennung die wäßrige Phase mit konzentrierter HCl auf einen pH-Wert von 2 angesäuert. Das sich abscheidende Öl wird abgetrennt, die Wasserphase mit Chloroform extrahiert, die organischen Extrakte vereinigt, getrocknet und eingeengt. Der Rückstand wird im Wasserstrahlvakuum fraktioniert. Bei einem Siedepunkt von 87° C/11 Torr gehen 226,1 g (81,2% d. Th.) 2,4,4-Trimethyl-3-oxopentannitril über.

## Beispiel 10

### 4,4-Dimethyl-2-ethyl-3-oxopentannitril

58 g (0,5 Mol) Pivalinsäuremethylester, 30 g (1 Mol) 80gew.-%iges NaH und 69 g (1 Mol) n-Butyronitril werden in Toluol, wie in Beispiel 9 beschrieben, umgesetzt. Nach Aufarbeitung wird das erhaltene Rohprodukt im Wasserstrahlvakuum fraktioniert. Bei einem Siedepunkt von 98—99°C/15 Torr gehen 50 g (65% d. Th.) 4,4-Dimethyl-2-ethyl-3-oxopentannitril über.

Analyse: $C_9H_{15}NO$ (153,2)

| | | | |
|---|---|---|---|
| Ber.: | C 70,5 | H 9,9 | N 9,14 |
| Gef.: | C 70,48 | H 10,12 | N 9,18 |

$^1H-NMR$ ($CDCl_3$):
$\delta$=3,83 (t, 1H) CO—CH; 1,91 (q, 2H) $CH_2-CH_3$; 1,23 (S, 9H) $C(CH_3)_3$; 1,06 ppm (t, 3H) $CH_2-CH_3$.

## Beispiel 11

### 4,4-Dimethyl-2-phenyl-3-oxopentannitril

116 g (1 Mol) Pivalinsäuremethylester, 60 g (2 Mol) Natriumhydrid (80gew.-%ige Suspension in Weißöl) und 234,3 g (2 Mol) Benzylcyanid werden in 750 ml trockenem Toluol bei 60°C bis zum Ende der Wasserstoffentwicklung umgesetzt. Zu der erkalteten Reaktionslösung werden 500 ml Wasser zugesetzt, ausgerührt und die wäßrige Phase nach der Abtrennung mit HCl auf pH 3 angesäuert und mit Chloroform extrahiert. Nach Einengen des Chloroforms wird der ölige Rückstand bei 0,6 Torr fraktioniert. Bei 111°C gehen 110,6 g (55% d. Th.) 4,4-Dimethyl-2-phenyl-3-oxopentannitril über.

Die spektroskopischen und analytischen Daten sind mit der Theorie übereinstimmend.

## Beispiel 12

### 4,4-Dimethyl-2-(3'-thienyl)-3-oxopentannitril

116 g (1 Mol) Pivalinsäuremethylester, 60 g (2 Mol) Natriumhydrid (80 Gew.-%) und 184,7 g (1,5 Mol) Thiophen-3-acetonitril werden, wie in Beispiel 8 beschrieben, in 750 ml trockenem Toluol umgesetzt. Die Reaktionszeit beträgt 24 Stunden. Das kristalline Rohprodukt wird zur Reinigung destilliert.

Bei einem Siedepunkt von 112°C/8.4 Torr erhält man 109 g (53% d. Th.) 4,4-Dimethyl-2-(3'-thienyl)-3-oxopentannitril, das einen Schmelzpunkt von 45—47°C besitzt.

Analyse: $C_{11}H_{13}NOS$ (207,3)

| | | | | |
|---|---|---|---|---|
| Ber.: | C 63,73 | H 6,32 | N 6,76 | S 15,47 |
| Gef.: | C 63,60 | H 6,34 | N 6,64 | S 14,90 |

$^1H-NMR$ ($CDCl_3$):
$\delta$=7,4—7,0 (m, 3H) $H_{Thiophen}$; 5,33 (S, 1H) CH—CN; 1,22 ppm (S, 9H) $C(CH_3)_3$.

## Beispiel 13

### 2-Benzoylpropionitril

137 g (1 Mol) Benzoesäuremethylester und 60 g (2 Mol) 80gew.-%iges Natriumhydrid werden in 750 ml Toluol auf 75°C erwärmt und bei dieser Temperatur innerhalb von 1,5 Stunden mit 110,2 g (2 Mol) Propionitril versetzt. Es wird bei 85—90°C bis zum Ende der Wasserstoffentwicklung gerührt. Die Reaktionsmischung wird abgesaugt und der Niederschlag in 1000 ml Wasser suspendiert und unter kräftigem Rühren mit Salzsäure auf pH 2 angesäuert. Das abgeschiedene Öl wird abgetrennt, die wäßrige Phase mit insgesamt 300 ml Toluol extrahiert. Die vereinigten organischen Phasen werden über Natriumsulfat getrocknet, eingeengt und das Öl im Vakuum fraktioniert.

Bei der Destillation erhält man 97 g (61% d. Th.) 2-Benzoylpropionitril vom Siedepunkt 110°C/0,6 Torr.

Analyse: $C_{10}H_9NO$ (159,1)

| | | | |
|---|---|---|---|
| Ber.: | C 75,45 | H 5,70 | N 8,80 |
| Gef.: | C 75,41 | H 5,89 | N 8,91. |

Beispiel 14

4,4-Dimethyl-2-(4'-chlorphenyl)-3-oxopentannitril

116 g (1 Mol) Pivalinsäuremethylester, 60 g (2 Mol) 80gew.-%iges Natriumhydrid und 151,5 g (1,5 Mol) 4-Chlorbenzylcyanid werden, wie in Beispiel 8 beschrieben, umgesetzt.

Nach Aufarbeitung erhält man 147,4 g (62,6% d. Th.) 4,4-Dimethyl-2-(4'-chlorphenyl)-3-oxopentannitril als viskoses Öl.

Analyse: $C_{13}H_{14}ClNO$ (235,45)

| | | | | |
|---|---|---|---|---|
| Ber.: | C 66,24 | H 5,97 | N 5,94 | Cl 15,05 |
| Gef.: | C 66,44 | H 6,06 | N 6,26 | Cl 15,74 |

$^1H-NMR$ ($CDCl_3$):

$\delta = 7{,}37$ (S, 4H) $H_{Ar}$; 5,20 (S, 1H) CH—CN; $1{,}20_{ppm}$ (S, 9H) $C(CH_3)_3$.

**Patentansprüche**

1. Verfahren zur Herstellung von 3-Oxonitrilen der allgemeinen Formel I,

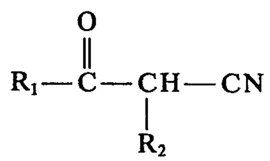

(I)

in der $R_1$ einen jeweils gegebenenfalls substituierten, tertiären Alkyl-, Cycloalkyl-, aromatischen oder heteroaromatischen Rest bedeutet, und $R_2$ für einen geraden oder verzweigten, gegebenenfalls substituierten Alkylrest mit 1 bis 4 C-Atomen, einen gegebenenfalls substituierten Aryl- oder Heteroarylrest, oder für ein Wasserstoffatom steht, durch Umsetzung eines Carbonsäureesters der allgemeinen Formel II,

$$R_1-COOR_3 \qquad (II)$$

in der $R_3$ für den Methyl- oder Äthylrest steht, mit einem Carbonsäurenitril der allgemeinen Formel III,

$$R_2CH_2CN \qquad (III)$$

wobei $R_1$ und $R_2$ die oben angegebenen Bedeutungen haben, in einem inerten Lösungsmittel in Gegenwart von Natriumhydrid und in Gegenwart eines Öles, dadurch gekennzeichnet, daß man das Natriumhydrid in Form einer 70—80%igen Suspension in Weißöl einsetzt, und dieses zusammen mit dem Ester gemäß der allgemeinen Formel II vorlegt.

2. Verfahren nach Anspruch 1, dadurch gekennzeichnet, daß man als inertes Lösungsmittel Toluol verwendet.

3. Verfahren nach den Ansprüchen 1 oder 2, dadurch gekennzeichnet, daß man das Natriumhydrid und den Ester der allgemeinen Formel II in einem Verhältnis von 1,5 bis 2,1 : 1, vorzugsweise von 2 : 1, einsetzt.

4. Verfahren nach den Ansprüchen 1 bis 3, dadurch gekennzeichnet, daß man das Nitril der allgemeinen Formel III und den Ester der allgemeinen Formel II in einem Molverhältnis von 1,0 bis 2,1 : 1, beziehungsweise von 1,5 bis 2,0 : 1, einsetzt.

5. Verfahren nach den Ansprüchen 1 bis 4, dadurch gekennzeichnet, daß man bei einer Temperatur zwischen 50 und 110°C, insbesondere zwischen 60 bis 100°C, vorzugsweise zwischen 80 und 95°C, umsetzt.

6. Verfahren nach den Ansprüchen 1 bis 5, dadurch gekennzeichnet, daß man in Gegenwart katalytischer Mengen eines Alkohols umsetzt.

## Claims

1. Process for the production of 3-oxonitriles corresponding to the general formula I,

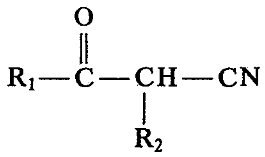

(I)

wherein, $R_1$ represents a tertiary alkyl-, cycloalkyl-, aromatic or heteroaromatic radical which is optionally substituted in each case, and $R_2$ represents a straight or branched, optionally substituted alkyl radical having from 1 to 4 C-atoms, an optionally substituted aryl or heteroaryl radical, or a hydrogen atom, by reacting a carboxylic acid ester corresponding to the general formula II,

$R_1-COOR_3$ (II)

wherein, $R_3$ represents a methyl or ethyl radical, with a carboxylic acid nitrile corresponding to the general formula III,

$R_2CH_2CN$ (III)

wherein, $R_1$ and $R_2$ are defined as above in an inert solvent in the presence of sodium hydride and in the presence of an oil, characterised in that the sodium hydride is used in the form of a 70 to 80% strength suspension in light oil, and this is used together with the ester corresponding to the general formula II as the starting solution.

2. Process according to claim 1, characterised in that toluene is used as inert solvent.

3. Process according to claims 1 to 2, characterised in that the sodium hydride and the ester corresponding to the general formula II are used in a ratio of from 1.5 to 2.1 : 1, preferably from 2 : 1.

4. Process according to claims 1 to 3, characterised in that the nitrile corresponding to the general formula III and the ester corresponding to the general formula II are used in a mol ratio of from 1.0 to 2.1 : 1, or from 1.5 to 2.0 : 1.

5. Process according to claims 1 to 4, characterised in that the reaction is carried out at a temperature of from 50 to 110° C, particularly from 60 to 100° C, preferably from 80 to 95° C.

6. Process according to claims 1 to 5, characterised in that the reaction is carried out in the presence of catalytic quantities of an alcohol.

## Revendications

1. Procédé pour la fabrication de 3-oxonitriles répondant à la formule générale I

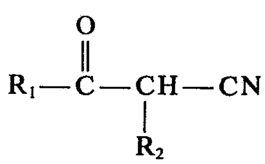

(I)

où $R_1$ représente un reste, éventuellement substitué, alcoyle tertiaire, cycloalcoyle, aromatique ou hétéroaromatique, et $R_2$ représente un reste alcoyle, linéaire ou ramifié, éventuellement substitué, à 1 à 4 atomes de C, un reste aryl ou hétéroaryl éventuellement substitué, ou un atome hydrogène, par réaction d'un ester d'acide carboxylique de formule générale II

$R_1-COOR_3$ (II)

où $R_3$ représente un radical méthyle ou éthyle, avec un nitrile d'acide carboxylique de formule générale III

$R_2CH_2CN$ (III)

où $R_1$ et $R_2$ ont les significations indiquées plus haut, dans un solvant inerte, en présence d'hydrure de sodium et en présence d'une huile, procédé caractérisé en ce que l'on met en œuvre l'hydrure de sodium sous la forme d'une suspension à 70 à 80% dans une huile blanche et qu'on met en place cet hydrure en commun avec l'ester de formule générale II.

2. Procédé suivant la revendication 1, caractérisé en ce que l'on utilise le toluène comme solvant inerte.

3. Procédé suivant les revendications 1 ou 2, caractérisé en ce que l'on met en œuvre l'hydrure de sodium et l'ester de formule générale II dans le rapport de 1,5 à 2,1 : 1 de préférence de 2 : 1.

4. Procédé suivant les revendications 1 à 3, caractérisé en ce que l'on met en œuvre le nitrile de formule générale III et l'ester de formule générale II dans un rapport moléculaire de 1 à 2,1 : 1 ou mieux de 1,5 à 2 : 1.

5. Procédé suivant les revendications 1 à 4, caractérisé en ce que l'on effectue la réaction à une température qui se situe entre 50 et 110° C, en particulier entre 60 et 100° C, ou mieux entre 80 et 95° C.

6. Procédé suivant les revendications 1 à 5, caractérisé en ce que l'on opère la réaction en présence de quantités catalytiques d'un alcool.